(19) Europäisches Patentamt
European Patent Office
Office européen des brevets

(11) Publication number: **0 219 207 B1**

(12) **EUROPEAN PATENT SPECIFICATION**

(45) Date of publication of patent specification: **01.07.92**

(51) Int. Cl.5: **A61K 47/30**, A61K 9/22, G02B 1/04

(21) Application number: **86306346.7**

(22) Date of filing: **15.08.86**

(54) **Sustained-release formulation comprising a hydrophobic polymer system.**

(30) Priority: **16.08.85 US 766735**

(43) Date of publication of application:
**22.04.87 Bulletin 87/17**

(45) Publication of the grant of the patent:
**01.07.92 Bulletin 92/27**

(84) Designated Contracting States:
**BE CH DE FR GB IT LI NL SE**

(56) References cited:
**EP-A- 140 828**
**EP-A- 0 124 017**
**FR-A- 2 365 606**
**GB-A- 1 391 438**
**US-A- 3 408 429**
**US-A- 3 618 604**
**US-A- 3 786 812**
**US-A- 3 831 604**
**US-A- 4 414 375**

(73) Proprietor: **BAUSCH & LOMB INCORPORATED**
**1400 North Goodman Street**
**Rochester, NY 14602(US)**

(72) Inventor: **Bawa, Rajan**
**7 Teakwood Lane**
**Fairport New York 14450(US)**

(74) Representative: **Allam, Peter Clerk et al**
**LLOYD WISE, TREGEAR & CO. Norman House 105-109 Strand**
**London WC2R 0AE(GB)**

**EP 0 219 207 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates generally to the preparation of a polymeric dosage form which gradually releases a medicinal agent therefrom. In specific embodiments, the invention pertains to the preparation of a formulation for slowly dispensing a drug in the eye.

It is basically known in the art that medicinally active substances may be dissolved in the aqueous constituent of hydrogels to gradually release such substances over an extended period. For example, US-A-3220960 describes utilizing a hydrogel in the eye as a carrier for time release medicaments such as boric acid or penicillin. Similarly, US-A-3551556, US-A-3641237, US-A-4003991 and US-A-4271143 disclose slowly releasing an active ingredient from an insoluble, cross-linked hydrogel in one form or another. Several compositions illustrated in the latter two patents are comprised of viscous, long-acting gel preparations where the prolongation of biological activity of the ophthalmic drug results from a slow erosion of the gel surface. The formulation in US-A-3551556 shows granular non-ionogenic, neutral, insoluble hydrogels which are useful for oral or intramuscular application. Further, many patents are directed to ocular insert devices which prolong the effect of a drug incorporated within the device. Such patents include US-A-3618604, US-A-3811444, US-A-3826258 and US-A-3786812.

These prior carriers of medicaments present certain difficulties during their use, particularly with ophthalmic drugs. The predominant complaint with long-acting gel formulations is blurred vision. Another difficulty is the inability to wear corrective contact lenses when a viscous material will be instilled and remain in the eye over an extended period of time. The ocular insert devices also present certain disadvantages with their use. When inserted into the conjunctival sac, such devices create a strong foreign body sensation and discomfort for the patient. The insert devices must be changed weekly. Additionally, the devices tend to fall out of the eye easily and cannot be used further by the patient since they are not capable of being sterilized.

Similarly, conventional contact lenses containing a sustained-release medicine carrier have drawbacks in practice. They have been found to obtain inadequately controlled or prolonged release characteristics making the conventional lenses unsuitable and impractical as sustained-release devices. The concept of soaking a high water content material in a drug solution has been used with conventional hydroxyethyl methacrylate based contact lenses, for example, a polymerized hydrophilic monomer or soft contact lens such as Soflens® manufactured by Bausch & Lomb. See Ruben et al, British J. Ophthal., 59:455 (1975). In practice, Soflens®, however, provides an inefficient system and is an unsuitable device for prolonged release. Experimental studies have shown that Soflens® will release 100% of pilocarpine hydrochloride in buffered saline and distilled water in merely $1\frac{1}{2}$ and $2\frac{1}{2}$, hours, respectively.

EP-A-140828 describes a polymeric dosage form for oral delivery of a medicinal agent prepared by imbibing a preformed copolymer with the medicinal agent. The copolymer which is used comprises a cross-linked copolymer of a hydrophobic monomer with optionally a hydrophilic monomer.

The present invention, in contrast, provides a process for the preparation of a polymeric dosage form of a medicinal agent useful for topical, systemic or transdermal administration, which process comprises the steps of:

(a) providing a liquid monomeric mixture which comprises:

(i) 20-90% of one or more hydrophobic monomers,

(ii) optionally up to 15% of one or more hydrophilic monomers,

(iii) 2-40% cross-linking agent,

(iv) 0.05 to 3% free-radical catalyst, and

(v) said medicinal agent in particulate form suspended in said mixture, said percentages being by weight based on the weight of the total monomers present, and

(b) polymerizing said monomeric mixture to obtain said polymeric dosage form.

The hydrophobic monomer component used in the polymer of this invention can be present in varying amounts, in the range from 20% to 90% w/w of the total monomers present. More preferably the hydrophobic monomer(s) are present in an amount of approximately 60% w/w of the total monomers present.

Among others, hydrophobic monomers which are useful as modulus modifiers can be used herein. The modulus modifier may be, for example, cycloalkyl ester, tertiary-butyl styrene, polycyclic acrylate or methacrylate, as well as mixtures thereof. More particularly the polycyclic modifiers may be isobornyl acrylate, isobornyl methacrylate, dicyclopentanedienyl acrylate, dicyclopentanedienyl methacrylate, adamantyl acrylate, adamantyl methacrylate, isopinocamphyl acrylate and isopinocamphyl methacrylate and mixtures thereof. The cycloalkyl ester modifier is of formula I below. Illustrative of these cycloalkyl modifiers are methyl methacrylate, menthyl acrylate, tertiary-butyl cyclohexyl methacrylate, isohexyl cyclopentyl

acrylate and methylisopentyl cyclooctyl acrylate.

(I) 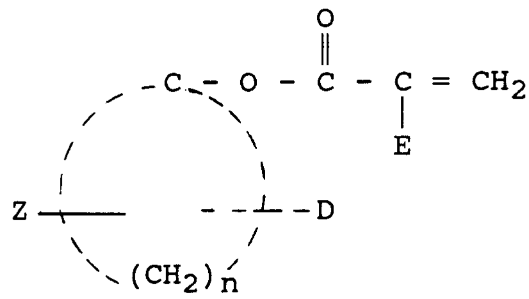

wherein:
D is branched or normal alkyl of 3 to 6 carbon atoms
E is H or $CH_3$
Z is H or $CH_3$
n is an integer from 3 to 8.

In addition to the modulus modifiers mentioned above, other well known hydrophobic monomers may be used in the formulation of the polymers and copolymers to tailor the properties to the particular application. For example, the hydrophobic monomers of this invention include monomers which contain at least one silicon or fluorine atom as a part of its composition. Useful hydrophobic monomers also include alkyl, cyclo-alkyl and aryl acrylates and methacrylates such as tertiary-butylmethacrylate, methyl methacrylate, ethyl methacrylate, cyclohexane methacrylate, hexyl methacrylate, isobornyl methacrylate, and methyl cyclohexyl methacrylate. Useful hydrophobic monomers further include mono- or disubstituted itaconates, styrene and its derivatives, acrylonitrile, vinyl esters such as vinyl acetate or vinyl pentaacetyl gluconate, vinyl ethers such as vinyl butyl ether, allyl esters such as allyl acetate, propionate or butyrate, fluorine containing monomers such as octafluoropentyl methacrylate and silicon containing monomer, e.g., methacryloxypropyl tris (trimethylsiloxy) silane or heptamethyltrisiloxanyl ethyl acrylate.

For some applications the polymerizates formed from the above hydrophobic monomer(s) and cross-linking agent(s) may lack the desired physical handling properties. In such circumstances it may be advantageous to incorporate up to 15% w/w of one or more hydrophilic monomers in the above polymers, desirably 2 % to 15 % w/w and, more preferably, approximately 8% w/w, based on of the total monomers present in the polymerization mixture. These monomers have an olefinic bond. They include, for example the hydroxyalkyl esters and amides, both N-substituted and unsubstituted, of alpha-, beta-unsaturated carboxylic acids, N-vinyl lactams and 2-acrylamido-2-methylpropane sulfonic acid. The alpha-, beta-unsaturated acids useful in this connection are acrylic acid, crotonic acid, methacrylic acid, itaconic acid, maleic acid, maleic anhydride and fumaric acid. The poly-functional alcohols which form the hydroxyalkyl esters include glycol, glycerol, propylene glycol, trimethylene glycol and other polyhydric alkanols, dialkylene glycols of 2 to 12 carbon atoms and polyalkylene glycols. Polyalkylene glycols are exemplified by triethylene glycol, tetraethylene glycol, pentaethylene glycol and hexaethylene glycol. However, the hydrophilic monomers are the alpha-, beta- unsaturated carboxylic acids themselves, such as methacrylic acid.

Useful amides of the foregoing acids include diacetone acrylamide and N-mono-substituted diacetone acrylamide. Also useful are the amines of the foregoing acids such as mono- or di-alkylamino substituents.

A nitrogen containing monomer which may be used as a hydrophilic monomer in the preparation of the copolymers is conveniently referred to as N-vinyl lactam and includes (a) N-vinyl lactams per se and (b) other heterocyclic N-vinyl monomers. Illustrative of the N-vinyl lactams that may be employed in this invention are N-vinyl-2-pyrrolidinone N-(1-methyl vinyl) pyrrolidinone, N-vinyl-2-piperidone and N-Vinyl-2-caprolactam, which may be substituted in the lactam ring by one or more lower alkyl groups such as methyl, ethyl or propyl, e.g., N-vinyl-5-methyl pyrrolidinone, N-vinyl-3,3-dimethyl pyrrolidinone, N-vinyl-5-ethyl pyrrolidinone and N-vinyl-6-methyl piperidone. Illustrative of the other heterocyclic N-vinyl monomers used in preparing the polymers of this invention are N-vinyl imidazole, N-vinyl succinimide, N-vinyl diglycolylimide, N-vinyl glutarimide, N-vinyl-3-morpholinone and N-vinyl-5-methyl-3-morpholinone. The lactams may be effectively employed alone or in admixture with other lactam monomers.

Optionally, an inhibitor or stabilizer may be added to the monomer mix if desirable. Suitable agents include 1,4-p-toluidiro anthraquinone, butylated hydroxytoluene (BHT), tertiarybutylcatecol and other quinone or hydroquinone types.

The monomers used in this invention are cross-linked by all types of cross-linking compounds used in

the prior art, see for instance, US-A-3,822,089; US-A-4,152,508; and US-A-4,440,919. The cross-linking agent can be employed in varying amounts in the range from 2 % to about 40 % w/w, preferably 23.5 % w/w, of the total monomers present. Examples of cross-linking agents include polyfunctional derivatives of the previously enumerated alpha-, beta-unsaturated acids, e.g., acrylic acid, methacrylic acid, crotonic acid, itaconic acid, maleic acid, fumaric acid, acrylamide, methacrylamide, and multi-vinyl substituted benzenes. More particularly these cross-linking agents include the following: ethylene glycol diacrylate or dimethacrylate, diethylene glycol diacrylate or dimethacrylate, triethylene glycol diacrylate or dimethacrylate, tetraethylene glycol diacrylate or dimethacrylate, polyethylene glycol diacrylate or dimethacrylate, trimethylolpropane triacrylate or trimethacrylate, bisphenol A diacrylate or dimethacrylate, ethoxylated bisphenol A diacrylate or dimethacrylate, pentaerythritol tri- and tetra-acrylate or methacrylate, tetramethylene diacrylate or dimethacrylate, methylene bisacrylamide or methacrylamide, dimethylene bisacrylamide or methacrylamide, N,N'-dihydroxyethylene bisacrylamide or methacrylamide, hexamethylene bisacrylamide or methacrylamide, decamethylene bisacrylamide or methacrylamide, divinyl benzene, vinyl methacrylate, and allyl methacrylate.

Still other useful cross-linking agents include 1,3-bis (4-methacryloyl oxyalkyl) tetra disiloxane and similar poly (organo-siloxane) monomers set forth in US-A-4,153,641. Another group of useful cross-linking agents are the resonance free di(alkylene tertiary amine) cyclic compounds e.g., N,N'-divinyl ethylene urea, as disclosed in US-A-4,436,887. Yet another group are di- or polyvinyl ethers of di- or polyvalent alcohols such as ethylene glycol divinyl ether.

The monomeric mixtures, comprising the hydrophobic monomer(s), the optional hydrophilic monomer(s) and a cross-linking agent, are generally clear liquids of varying viscosity. These monomer mixtures can be readily cured to cast shapes by conventional methods such as free radical initiation.

As catalysts for carrying out the polymerization, there are employed a free radical catalyst (initiator) in an amount from 0.05 % to 3 % w/w of the polymerizable monomer mixture. The preferred amount of catalyst is 0.1% to 0.5% w/w of the total monomers present. Usually, the catalyst is added initially to the monomers and then the polymerization procedure is completed. The free radical type catalysts suitable for this invention include peroxides, azo compounds, oxidation-reduction systems and similar catalysts described in the literature. Typical catalysts include dimethyl 2,2'-azobis [isobutylate], benzoin methyl ether, tertiary-butyl-peroctoate, benzoyl peroxide, isopropyl percarbonate, methyl ethyl ketone peroxide, cumene hydroperoxide, dicumyl peroxide, bis(isopropyl) peroxydicarbonate, 2,2'-azobis[isobutyronitrile], acetyl peroxide, lauroyl peroxide, decanoyl peroxide, 2,2'-azobis[2,4-dimethylvaleronitrile], 2,2'-azobis [2,4-dimethyl-4-methoxyvaleronitrile], phthalic peroxide, diethoxyacetophenone, and tertiary-butyl peroxypivalate. Polymerization generally can be effected at 20 to 150°C, usually 40 to 90°C, and visible light or irradiation, eg ultraviolet light or gamma rays, may also, if desired, be used to initiate the polymerization.

Using methods well known in the art, the polymeric dosage form of this invention can be formed into a variety of shapes depending upon the end use and desired results to be obtained therefrom. For ophthalmic purposes, the polymeric matrix can have any form to maintain direct contact with the eye. It is not necessary to cover the entire cornea if the dosage form is merely used to instill a continuous flow of an ophthalmic drug in the eye. If the dosage form will also be used to correct vision, then it may be desirable to cast the polymer on an optical mold.

By way of example, the mixture of hydrophobic monomer(s), cross-linking agent, free radical catalyst and optional hydrophilic monomer(s) described above is purged with an inert gas such as nitrogen or carbon dioxide and filled into polypropylene tubes having dimensions of 18mm x 300 mm. The polymerization is then carried out by gradually heating from 30°C to 110°C in a step fashion over a span of several days. In a typical schedule the tubes are placed in a water bath from 30°C to 50°C for two to three days followed by two days at 60°C. The rod is then removed from the mold and post-cured at 110°C for a period of about four hours. The fully cured rods are then cut into cylinders, optionally then annealed at temperatures up to 150°C and machined to form contact lenses desired. A spin-casting process as described in US-A-3,408,429 and US-A-3,496,254 can be employed to prepare useful objects in accordance with this invention. The manufacturing technique may involve triple spin-casting or a casting method that does not involve spinning. Such static casting optionally may use polypropylene domes for lens shape and easy detachment from the lens surface. Of course, any conventional material which can give a proper lens shape would suffice.

This invention contemplates a variety of other processes for preparing the polymeric dosage form whereby the medicinal agent is retained by the polymeric matrix and, upon tissue contact, is gradually released into the tissue. One process comprises incorporating the medicament directly into the monomer mixture and polymerizing the monomers in the presence of the drug to make a single layer of polymer plus drug as the dosage form. Another process uses the drug-polymer layer as a middle layer in a "sandwich"

dosage form in which two drug-free polymer layers are formed on either side of the middle layer containing the drug. Alternatively, a two layer system may be formed having one layer as polymer plus drug and the other layer as drug-free polymer.

In any of the above methods of preparation, the medicament is incorporated as a suspension of drug particles in the monomer solution mix before it is polymerized. The amount of the drug used in the preparation of these dosage forms will vary depending upon the physicochemical properties of the selected drug and the therapeutic effect desired to be achieved. As an example of pilocarpine hydrochloride, varying concentrations of the compound may be employed, desirably an amount from 0.1% to 50 % w/w, and preferably 10 % w/w, for incorporation in the dosage form of choice.

To prepare the final dosage form as a contact lens, it is desirable to use a combination of spin or static casting on one surface and a lathing technique on the other surface obtaining the drug particles in the periphery of the lens away from the optical zone. After lathing, the particles form a ring around the center of the lens. However, any procedure known in the art which keeps the drug particles in the periphery and maintains the optical zone of the lens clear of the particles would suffice.

Regardless of the exact mode of preparation, the polymeric composition provides appreciable time release of the medicament. The release rate varies with cross-linking density, type of polymer barrier system and mode of preparation. For example, a layering technique where only a middle layer contains polymer plus drug may provide a slower release profile than a single layer of polymer plus drug.

To quantify the release rates, lenses with and without drug can be placed in a known quantity of release media (distilled water or buffered saline) and stirred with a magnetic stirrer. At various times the lenses can be transferred to fresh media and the absorbance of the previous media can be determined by ultraviolet spectroscopy. The absorbance of the media containing drugged lenses is reduced by the absorbance of the media containing undrugged lenses. Use of a calibration curve relating absorbance to concentration allows for a determination of the concentration of the drug. The calibration curve is developed by measuring the absorbance of known concentrations of the drug in the release media. As the concentrations ($\mu$g/ml) of the drug and the volume (ml) of release media are known, the amount of the released drug can be calculated ($\mu$g). This value divided by the time of exposure to the media gives the release rate in $\mu$g/hr which is plotted against time.

Contact lenses made according to the instant invention must be oxygen permeable. A critical oxygen tension and flux under a lens should be about 10 mm Hg and 2 $\mu$l/(cm$^2$hr.) respectively below which corneal swelling occurs, see Polse and Decker, Investigative Ophthalmology and Visual Science, 18:188 (1979). In order to meet these requirements, the lens material must have adequate oxygen permeability. The contact lenses of this invention should desirable have an oxygen permeability of at least about 20x10$^{-11}$ cm$^3$cm/(sec.cm$^2$mmHg), as well as being hydrolytically stable, biologically inert and transparent.

An article formed from the disclosed polymers and copolymers may be used to administer medications to mammals where an article compatible with living tissue or with the mucous membranes is desired. The term "biomedical devices" means the materials disclosed herein have nontoxic, nonirritating physiochemical properties rendering them suitable for prolonged contact with living tissue, blood and the mucous membranes. It is known that blood, for example, is rapidly damaged in contact with artificial surfaces. The design of a synthetic surface which is antithrombogenic and nonhemolytic to blood is necessary for drug administering devices used with blood, especially an implanted or transdermal device. The polymers and copolymers employed for the purposes of the instant invention are compatible with living tissue. Sterilization can be achieved by gamma-irradiation, ultraviolet light irradiation, ethylene oxide exposure and other well known techniques for hydrophobic polymers.

The instant invention provides a polymeric dosage form useful for topical, systemic or transdermal administration of a medicinal agent which comprises above-described polymeric matrix in association with a therapeutically effective amount of the medicinal agent. The specific hydrophobic polymeric matrix permits the subsequent release of the active ingredient at a gradual, carefully controlled rate prolonging the time release period. In contrast to normal disintegration time, the sustained-release administration of medicaments enhances therapeutic effectiveness and decreases many undesirable side effects by maintaining constant tissue and blood levels.

The polymeric dosage form can be used in preparing biomedical devices that upon surgical implant will provide sustained-release activity of the active ingredient. Depending on the location of the implant, the therapeutic effect may be local or systemic. The dosage form can also provide for the oral or topical (i.e., localized activity on the skin) controlled-release administration of medicaments. Additionally, the dosage form can be utilized for the transdermal controlled-release administration of medicaments, i.e., the device is retained in contact with the skin for transdermal absorption of the medicament into the blood for systemic effect. Contact with the skin may be achieved by any means well-known in the art such as by incorporating

an adhesive in or on the device, or by adhering the device within or onto a bandage-type article.

Further, the dosage form can be useful for the ophthalmic route of administering medicinal agents for either local or systemic therapeutic effect. The term "ophthalmic administration of a systemic medicament" defines the administration of a medicinal agent to a mammal by the ophthalmic route whereby the medicinal agent acts therapeutically to create a systemic effect. For ophthalmic administration of a medicament to produce local or systemic activity, the polymeric composition can be molded into any convenient shape for eye contact. If correcting vision is not necessary, the dosage form does not have to cover the entire cornea. Alternatively, the polymeric matrix can be shaped into contact lenses on optical molds if it is desirable to correct vision in addition to administer ophthalmic medicaments.

The length of time for contacting the polymeric device containing a medicament with a tissue of a mammal depends upon the individual circumstances of each case. A sufficient amount of time to achieve a constant therapeutic effect varies in accordance with the optimum therapeutic effect desired. The duration of therapy is, of course, contingent upon the disease or medical problem being treated or palliated. Likewise, the therapeutically effective amount of the specific medicinal agent is determined by therapy requirements and the biophysical properties of the active compound. For example, with respect to contact lenses, the invention contemplates daily wear or extended wear, typically, up to a month. To treat an eye infection, it would be desirable to maintain the contact lens containing an antibiotic or an antiviral agent on the eye for one to two weeks. On the other hand, to treat glaucoma, it would be desirable to wear the lens containing an agent for reducing intraocular pressure for the maximum time that extended wear contact lenses can remain in the eye.

The term "dosage form" refers to physically discrete units suitable as unitary dosage for mammals, each unit containing a predetermined quantity of active component calculated to produce the desired therapeutic effect.

The term "a medicinal agent" means a substance used in treating or ameliorating a disease or medical condition. For purposes of this invention, "a medicinal agent" refers to drugs which would have the capacity to be bound to the hydrophobic polymeric matrix. That is, any drug or its salts with polar characteristics could be used in the present invention. Examples of medicinal agents include antibiotics, antivirals, anti-inflammatories, steroids, peptides, polypeptides, cardiotonics, antihypertensives, antiallergics, alpha-and beta-adrenergic blocking agents, anticataract agents, ophthalmic medicaments, ophthalmic lubricating agents, ophthalmic topical or regional anesthetic agents. The ophthalmic medicaments or other medicinal agents encompass such drugs as pilocarpine, idoxuridine, carbachol, bethanechol, timolol, tetracycline, epinephrine, phenylephrine, eserine, phospholine, demecarium, cyclopentolate, homatropine, scopolamine, nitroglycerin, chlortetracycline, bacitracin, neomycin, polymyxin, gramicidin, oxytetracycline, chloramphenicol, gentamycin, penicillin, erythromycin, sulfacetamide, polymyxin B, tobramycin, isofluorophate, fluoromethalone, dexamethasone, hydrocortisone, fluorocinolone, medrysone, prednisolone, methyl prednisolone, betamethasone, triamcinolone, interferon, cromolyn, all-*trans* retinoic acid (Vitamin A), and the nontoxic, pharmaceutically acceptable salts thereof. The category of ophthalmic lubricating agents refers to those agents capable of inducing natural lacrimation or creating artificial lacrimation and includes, for example, polyvinyl alcohol, cellulose polymers such as hydroxypropyl methyl cellulose, a polylactam such as polyvinyl pyrrolidinone and other tear inducers or substitutes. The topical or regional anesthetic agents, which may be useful during ophthalmic surgery or other ophthalmic procedures, include lidocaine, cocaine, benoxinate, dibucaine, proparacaine, tetracaine, etidocaine, procaine, hexylcaine, bupivacaine, mepivacaine, prilocaine and chloroprocaine.

The term "pharmaceutically acceptable salt" refers to those salts of the parent compound which do not significantly or adversely affect the pharmaceutical properties (*e.g.*, toxicity, efficacy,) of the parent compound. The salts of the present invention which are pharmaceutically acceptable include, for example, chloride, iodide, bromide, hydrochloride, acetate, nitrate, stearate, phosphate and sulfate. It is desirable to use the appropriate salt form of the active ingredient which would increase the water solubility or polar characteristics of the base compound.

The following examples illustrate the present invention. All parts and percents referred to herein are on a weight basis and all temperatures are expressed in degrees Celsius unless otherwise specified. Room temperature refers to about 23°C to about 28°C. The examples were all conducted at atmospheric pressure.

## Example 1

### Preparation of a Single Layer Hydrophobic Polymer Containing Medicament

A monomer mix solution is prepared by mixing together 42 g of methacryloxypropyl tris-(trimethylsiloxy)silane, 23.5 g of triethylene glycol dimethacrylate, 21 g of cyclohexane methacrylate 8 g of methacrylic acid, 5 g of methyl methacrylate, 0.49 g of dimethyl 2,2'-azobis [isobutylate] and 0.01 g of 1,4-p-toluidiro anthraquinone.

In a vial, 0.2 g of pilocarpine hydrochloride is weighed. Using a dropper, 2 g of the monomer mix solution is added to the vial of pilocarpine hydrochloride. A magnetic stirrer is dropped in the vial and the vial is capped. The contents of the vial are stirred vigorously using a magnetic stir plate for one hour. A suspension of drug particles dispersed throughout the hydrophobic monomer mix is achieved. The mix is polymerized by ultraviolet light (UV) at room temperature under nitrogen for 20 minutes and post-cured for 30 minutes under UV at 70 °C under nitrogen, against a suitable optical mold.

Example 2

Preparation of Single-Layer Hydrophobic Polymer Containing Medicament

In a beaker, 10 g of pilocarpine hydrochloride is weighed. To that is added 100 g of the monomer mix solution of Example 1. A high shear mixer is used for about 20 minutes to wet-mill the drug particles in the mix to fine particle size. The mix is polymerized according to the procedure in Example 1.

Example 3

Preparation of Triple-Layer Hydrophobic Polymer Containing Medicament

A polymer without drug is prepared against a suitable mold from the same monomer mix of Example 1 and is cured by UV at room temperature under nitrogen until it is a semi-solid without post-curing. The same procedure as described in Example 1 is followed to cast a layer of polymer plus pilocarpine hydrochloride on top of the polymer. The same procedure is again repeated to cast a third layer without the drug on top.

Example 4

Preparation of Contact Lens

The procedure of Example 3 is repeated to prepare the hydrophobic polymer containing no drug as a first layer. As a second layer, the drug-containing suspension of Example 1 is cured on top of the polymer by UV at room temperature under nitrogen for 20 minutes and post-cured for 30 minutes under UV at 70 °C under nitrogen. The films are lathed to obtain a contact lens with the drug particles in the periphery of the lens.

Example 5

Release Studies I

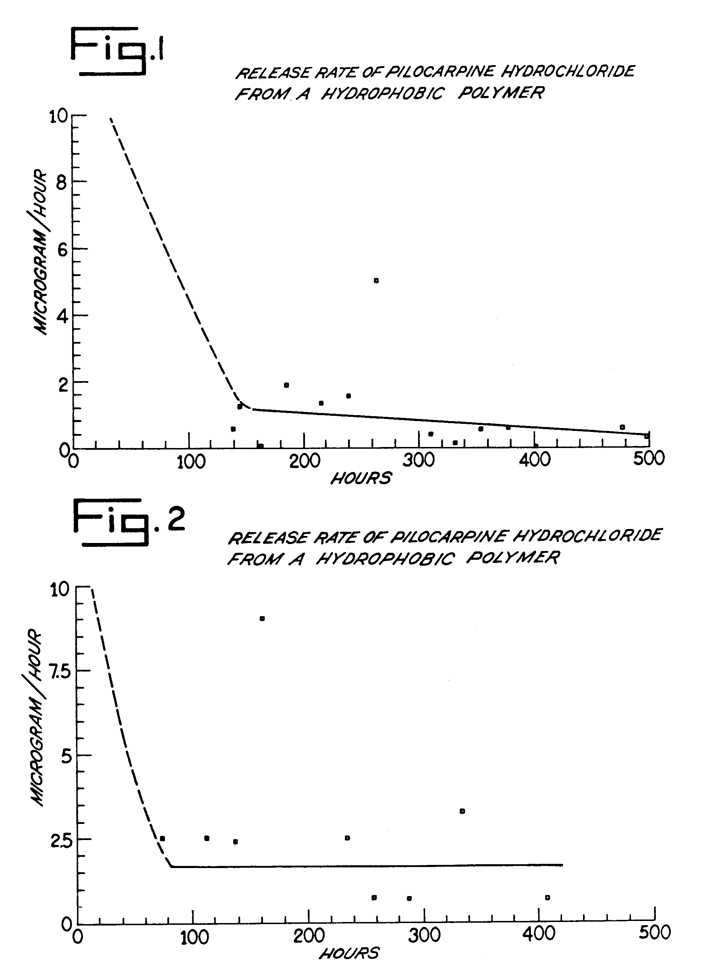

The procedure of Example 2 is repeated to prepare a single-layer hydrophobic polymer containing pilocarpine hydrochloride. The polymer is placed in buffered saline with stirring. With appropriate controls, the release rate of the drug is measured by UV spectroscopy at 215 nm. The results are shown in Figure 1.

Example 6

Release Studies II

The procedure of Example 3 is repeated to prepare a triple-layer hydrophobic polymer containing pilocarpine hydrochloride. The polymer is placed in buffered saline and the release rate is measured. The results are shown in Figure 2.

## Claims

**1.** A process for the preparation of a polymeric dosage form of a medicinal agent useful for topical,

systemic or transdermal administration, which process comprises the steps of:

(a) providing a liquid monomeric mixture which comprises:

(i) 20-90% of one or more hydrophobic monomers,

(ii) optionally up to 15% of one or more hydrophilic monomers,

(iii) 2-40% cross-linking agent,

(iv) 0.05 to 3% free-radical catalyst, and

(v) said medicinal agent in particulate form suspended in said mixture, said percentages being by weight based on the weight of the total monomers present, and

(b) polymerizing said monomeric mixture to obtain said polymeric dosage form.

**2.** A process according to Claim 1, wherein the monomeric mixture comprises a hydrophobic monomer having properties as a modulus modifier.

**3.** A process according to Claim 2, wherein the modulus modifier is a polycyclic acrylate or methacrylate, preferably cyclohexane methacrylate.

**4.** A process according to any preceding claim, wherein the monomeric mixture comprises from 2-15% by weight, based on the weight of total monomers present, of hydrophilic monomer, preferably of an alpha-, beta-unsaturated carboxylic acid.

**5.** A process according to any preceding claim, wherein the monomeric mixture comprises 0.1 to 0.5% of free radical catalyst, based on the weight of total monomers present.

**6.** A process according to any preceding claim, wherein said medicinal agent is an antibiotic, an antiviral, an anti-inflammatory, a steroid, a peptide, a polypeptide, a cardiotonic, an antihypertensive, an antiallergic, an alpha-adrenergic blocking agent, a beta-adrenergic blocking agent, an anti-cataract agent, an ophthalmic medicament, an ophthalmic lubricating agent or an ophthalmic anaesthetic agent.

**7.** A process according to any preceding claim, wherein said medicinal agent is selected from pilocarpine, idoxuridine, carbachol, bethanechol, timolol, tetracycline, epinephrine, phenylephrine, eserine, phospholine, demecarium, cyclopentolate, homatropine, scopolamine, nitroglycerin, chlortetracycline, bacitracin, neomycin, polymyxin, gramicidin, oxytetracycline, chloramphenicol, gentamycin, penicillin, erythromycin, sulfacetamide, polymyxin B, tobramycin, isofluorophate, fluoromethalone, dexamethasone, hydrocortisone, fluorocinolone, medrysone, prednisolone, methyl prednisolone, betamethasone, triamcinolone, interferon, cromolyn, all trans-retinoic acid and the nontoxic, pharmaceutically acceptable salts thereof.

**8.** A process according to Claim 6, wherein said medicinal agent is an ophthalmic lubricating agent selected from polyvinyl alcohol, a cellulose polymer and a polylactam.

**9.** A process according to any preceding claim, wherein said monomeric mixture is polymerized in situ on a polymer layer previously formed by polymerizing a second liquid monomeric mixture which comprises:

(i) 20-90% of one or more hydrophobic monomers,

(ii) optionally up to 15% of one or more hydrophilic monomers,

(iii) 2-40% cross-linking agent, and

(iv) 0.05 to 3% free radical catalyst,

said percentages being by weight based on the weight of the total monomers of said second monomeric mixture,

whereby there is formed a two-layer polymeric dosage form with medicinal agent present in one layer.

**10.** A process according to Claim 9, wherein a third monomer mixture is polymerized in situ on the exposed layer of said medicinal agent-containing polymer layer, whereby there is formed a three-layer polymeric dosage form with medicinal agent present in the middle layer.

**11.** A process according to Claim 9, wherein said polymerizations are effected in an optical mold, and wherein the process comprises a subsequent lathing step to form a contact lens with a clear optical

zone and the medicinal agent in the periphery of the lens.

**Revendications**

1. Procédé de préparation d'une forme d'administration polymérique d'un agent médicinal utile pour l'administration topique, systémique ou transdermale, lequel procédé comprend les étapes consistant à :
   a) obtenir un mélange de monomères liquide qui comprend:
      1) 20 à 90 % d'un ou plusieurs monomères hydrophobes;
      2) facultativement jusqu'à 15 % d'un ou plusieurs monomères hydrophiles;
      3) 2 à 40 % d'agent réticulant;
      4) 0,05 à 3 % de catalyseur à radicaux libres, et
      5) ledit agent médicinal en forme particulaire en suspension dans ledit mélange, lesdits pourcentages s'entendant en poids et basés sur le poids total des monomères présents, et
   b) polymériser ledit mélange de monomères pour obtenir ladite forme d'administration polymérique.

2. Procédé selon la revendication 1, dans lequel le mélange de monomères comprend un monomères hydrophobe ayant des propriétés lui faisant jouer le rôle de modificateur de module.

3. Procédé selon la revendication 2, dans lequel le modificateur de module est un acrylate ou un méthacrylate polycyclique, de préférence le méthacrylate de cyclohexane.

4. Procédé selon l'une quelconque des revendications précédentes, dans lequel le mélange de monomères comprend 2 à 15 % en poids, rapporté au poids total des monomères présents, de monomères hydrophile, de préférence d'un acide carboxylique à insaturations alpha, bêta.

5. Procédé selon l'une quelconque des revendications précédentes, dans lequel le mélange de monomères comprend 0,1 à 0,5 % de catalyseur à radicaux libres, le pourcentage étant rapporté au poids total de monomères présents.

6. Procédé selon l'une quelconque des revendications précédentes, dans lequel ledit agent médicinal est un antibiotique, un antiviral, un anti-inflammatoire, un stéroïde, un peptide, un polypeptide, un cardiotonique, un anti-hypertensif, un anti-allergique, un agent bloquant alpha-adrénergique, un agent bloquant bêta-adrénergique, un agent anti-cataracte, un médicament ophtalmique, un agent lubrifiant ophtalmique ou un agent anesthésique ophtalmique.

7. Procédé selon l'une quelconque des revendications précédentes, dans lequel on choisit ledit agent médicinal parmi la pilocarpine, l'idoxuridine, le carbachol, le béthanechol, le timolol, la tétracycline, l'épinéphrine, la phényléphrine, l'ésérine, la phospholine, le démécarium, le cyclo-pentolate, l'homatropine, la scopolamine, la nitroglycérine, la chlorotétracycline, la bacitracine, la néomycine, la polymyxine, la gramicidine, l'oxytétracycline, le chloramphénicol, la gentamycine, la pénicilline, l'érythromycine, le sulfacétamide, la polymyxine B, la tobramycine, l'isofluorophate, la fluorométholone, la déxaméthasone, l'hydrocortisone, la fluorocinolone, la médrysone, la prednisolone, la méthylprednisolone, la bêtaméthsone, la triamcinolone, l'interféron, la cromolyne, tous acides trans-rétinoïque et leurs sels non-toxiques, pharmaceutiquement acceptables.

8. Procédé selon la revendication 6, dans lequel ledit agent médicinal est un agent lubrifiant ophtalmique choisi parmi l'alcool polyvinylique, un polymère cellulosique et un polylactame.

9. Procédé selon l'une quelconque des revendications précédentes, dans lequel on polymérise ledit mélange de monomères <u>in situ</u> sur une couche de polymère formée préalablement en polymérisant un second mélange de monomères liquide qui comprend :
   1) 20 à 90 % d'un ou plusieurs monomères hydrophobes;
   2) facultativement jusqu'à 15 % d'un ou plusieurs monomères hydrophiles;
   3) 2 à 40 % d'agent réticulant, et
   4) 0,05 à 3 % de catalyseur à radicaux libres,

   lesdits pourcentages s'entendant en poids sur la base du poids de la totalité des monomères dudit

second mélange de monomères,
de telle sorte qu'il se forme une forme d'administration polymérique à deux couches, l'agent médicinal étant présent dans l'une des couches.

**10.** Procédé selon la revendication 9, dans lequel on polymérise un troisième mélange de monomères in situ sur la couche exposée de ladite couche de polymère contenant l'agent médicinal, de telle sorte qu'on forme une forme d'administration polymère à trois couches, l'agent médicinal étant présent dans la couche centrale.

**11.** Procédé selon la revendication 9, dans lequel on effectue lesdites polymérisation dans un moule optique, et ledit procédé comprenant une étape de travail au tour subséquent pour former une lentille de contact avec une zone optique transparente et l'agent médicinal à la périphérie de la lentille.

**Patentansprüche**

**1.** Verfahren zur Herstellung einer zur topischen, systemischen oder transdermalen Verabreichung brauchbaren polymeren Dosierungsform eines Arzneimittels, bei dem:
(a) eine flüssige monomere Mischung vorgelegt wird, die:
(i) 20 - 90 % eines oder mehrerer hydrophober Monomere,
(ii) gegebenenfalls bis zu 15 % eines oder mehrerer hydrophiler Monomere,
(iii) 2 - 40 % Vernetzungsmittel,
(iv) 0,05 - 3 % freiradikalischen Katalysator und
(v) das teilchenförmige, in der Mischung suspendierte Arzneimittel
umfaßt, wobei die Prozentangaben Gewichtsprozente bezogen auf das Gewicht der insgesamt vorhandenen Monomere sind, und
(b) diese monomere Mischung polymerisiert wird, um die polymere Dosierungsform zu erhalten.

**2.** Verfahren nach Anspruch 1, bei dem die monomere Mischung ein hydrophobes Monomer mit Eigenschaften als Modulmodifizierungsmittel umfaßt.

**3.** Verfahren nach Anspruch 2, bei dem das Modulmodifizierungsmittel ein polycyclisches Acrylat oder Methacrylat, vorzugsweise Cyclohexylmethacrylat ist.

**4.** Verfahren nach einem der vorhergehenden Ansprüche, bei dem die monomere Mischung, bezogen auf das Gewicht der insgesamt vorhandenen Monomere, 2 - 15 Gew.-% hydrophiles Monomer, vorzugsweise eine $\alpha,\beta$-ungesättigte Carbonsäure, umfaßt.

**5.** Verfahren nach einem der vorhergehenden Ansprüche, bei dem die monomere Mischung 0,1 - bis 0,5 Gew.-% freiradikalischen Katalysator, bezogen auf das Gewicht der insgesamt vorhandenen Monomere, umfaßt.

**6.** Verfahren nach einem der vorhergehenden Ansprüche, bei dem das Arzneimittel ein Antibiotikum, ein Antivirusmittel, ein entzündungshemmendes Mittel, ein Steroid, ein Peptid, ein Polypeptid, ein Kardiotonikum, ein Antihypertonikum, ein Antiallergikum, ein $\alpha$-Blocker, ein $\beta$-Blocker, ein Antikataraktmittel, ein ophthalmisches Medikament, ein ophthalmisches Schmiermittel oder ein ophthalmisches Anästhetikum ist.

**7.** Verfahren nach einem der vorhergehenden Ansprüche, bei dem das Arzneimittel ausgewählt ist aus Pilocarpin, Idoxuridin, Carbachol, Bethanechol, Timolol, Tetracyclin, Epinephrin, Phenylephrin, Eserin, Phospholin, Demacarium, Cyclopentolat, Homatropin, Scopolamin, Nitroglycerin, Chlortetracyclin, Bacitracin, Neomycin, Polymyxin, Gramicidin, Oxytetracyclin, Chloramphenicol, Gentamycin, Penicillin, Erythromycin, Sulfacetamid, Polymyxin B, Tobramycin, Fluostigmin, Fluormethalon, Dexametason, Hydrocortison, Fluorocinolon, Medryson, Prednisolon, Methylprednisolon, Betamethason, Triamcinolon, Interferon, Cromolyn, all trans-Retinsäure und den nichttoxischen, pharmazeutisch akzeptablen Salzen derselben.

**8.** Verfahren nach Anspruch 6, bei dem das Arzneimittel ein ophthalmisches Gleitmittel ausgewählt aus Polyvinylalkohol, einem Cellulosepolymer und einem Polylactam ist.

**9.** Verfahren nach eine der vorhergehenden Ansprüche, bei dem die monomere Mischung in situ auf einer Polymerschicht polymerisiert wird, die zuvor durch Polymerisation einer zweiten flüssigen, monomeren Mischung gebildet worden ist, die:

(i) 20 - 90 % eines oder mehrerer hydrophober Momomere
(ii) gegebenenfalls bis zu 15 % eines oder mehrerer hydrophiler Monomere,
(iii) 2 - 40 % Vernetzungsmittel und
(iv) 0,05 - 3 % freiradikalischen Katalysator

umfaßt, wobei die Prozentangaben Gewichtsprozente bezogen auf das Gewicht der gesamten Monomeren der zweiten monomeren Mischung sind, wodurch eine zwei Schichten umfassende polymere Dosierungsform gebildet wird, die in einer Schicht Arzneimittel enthält.

**10.** Verfahren nach Anspruch 9, bei dem eine dritte Monomermischung in situ auf der freiliegenden Schicht der Arzneimittel enthaltenden Polymerschicht polymerisiert wird, wodurch eine drei Schichten umfassende polymere Dosierungsform gebildet wird, die in der Mittelschicht Arzneimittel enthält.

**11.** Verfahren nach Anspruch 9, bei dem die Polymerisierungen in einer optischen Form bewirkt werden und anschließend eine Drehbearbeitung erfolgt, um eine Kontaktlinse mit einer klaren optischen Zone und dem Arzneimittel in der Peripherie der Linse zu bilden.

Fig.1

RELEASE RATE OF PILOCARPINE HYDROCHLORIDE FROM A HYDROPHOBIC POLYMER

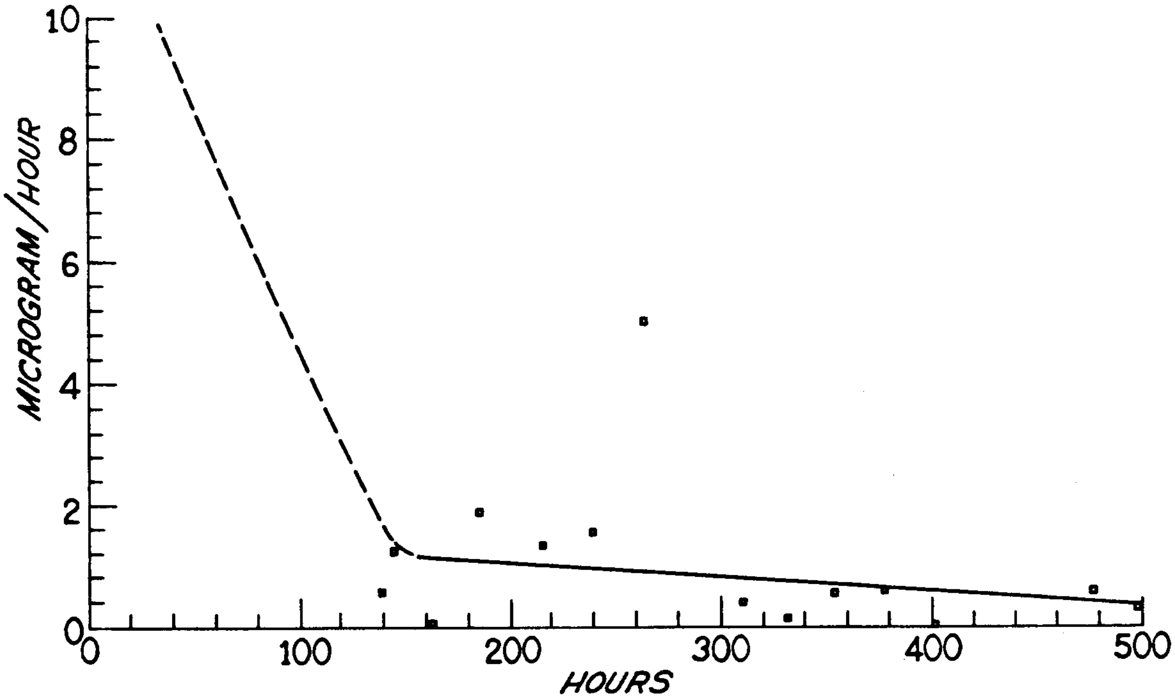


Fig.2

RELEASE RATE OF PILOCARPINE HYDROCHLORIDE FROM A HYDROPHOBIC POLYMER